# EUROPÄISCHE PATENTSCHRIFT

(11) **EP 3 085 248 B1**
(45) Veröffentlichungstag und Bekanntmachung des Hinweises auf die Patenterteilung: **01.07.2020**
(21) Anmeldenummer: 15166154.3
(22) Anmeldetag: 03.05.2015
(51) Int. Cl.: A23F 3/40, A61K 47/12, A61K 33/06, A23L 27/00, A23L 27/20

(54) **ZUBEREITUNGEN ENTHALTEND DEHYDROABIETINSÄURE**
COMPOSITIONS COMPRISING DEHYDRO ABIETIC ACID
COMPOSITIONS CONTENANT ACIDE DEHYDRO ABIETIQUE

(30) Priorität: 22.04.2015 WO PCT/EP2015/058741
(43) Veröffentlichungstag der Anmeldung: 26.10.2016
(73) Patentinhaber: Analyticon Discovery GmbH, 14473 Potsdam (DE); B.R.A.I.N. Biotechnology Research and Information Network AG, 64673 Zwingenberg (DE)
(72) Erfinder: Siems, Karsten, 14552 Michendorf (DE); Kluge, Grit, 14959 Trebbin (DE); Krohn, Michael, 64653 Lorsch (DE); Riedel, Katja, 64625 Bensheim (DE)
(74) Vertreter: Fabry, Bernd

(56) Entgegenhaltungen:
- JP-A- 2006 312 590
- US-A- 6 048 568
- US-A1- 2014 080 842
- US-A1- 2014 234 455
- US-A1- 2014 363 530
- VARGAS IRASEMA ET AL: "ANTIMICROBIAL AND ANTIOXIDANT COMPOUNDS IN THE NONVOLATILE FRACTION OF EXPRESSED ORANGE ESSENTIAL OIL", JOURNAL OF FOOD PROTECTION, INTERNATIONAL ASSOCIATION FOR FOOD PROTECTION, US, Bd. 62, Nr. 8, 1. August 1999 (1999-08-01) , Seiten 929-932, XP009080545, ISSN: 0362-028X
- MITANI ET AL: "Analysis of abietic acid and dehydroabietic acid in food samples by in-tube solid-phase microextraction coupled with liquid chromatography-mass spectrometry", JOURNAL OF CHROMATOGRAP, ELSEVIER, AMSTERDAM, NL, vol. 1146, no. 1, 8 March 2007 (2007-03-08), pages 61-66, XP005917998, ISSN: 0021-9673, DOI: 10.1016/J.CHROMA.2007.01.118

## Beschreibung

### GEBIET DER ERFINDUNG

Die Erfindung befindet sich auf dem Nahrungsmittelsektor und betrifft neue Zubereitungen mit Dehydroabietinsäure, Mittel die diese enthalten, Verfahren zur Geschmacksmodulierung sowie entsprechende Verwendungen der Stoffe. Die Erfindung ist durch die anhängigen Ansprüche definiert.

### STAND DER TECHNIK

In einer Zeit, in der Fitness und Gesundheit einen immer höheren Stellenwert gewinnen, hat es im Kauf- und Konsumverhalten von insbesondere jungen Verbrauchern einen erheblichen Wandel gegeben. Nachgefragt werden Produkte, die keinen Zucker enthalten, sondern Süßstoffe - seien sie synthetisch hergestellt wie etwa Saccharin oder Acesulfam K oder pflanzlicher Herkunft wie beispielsweise Stevia - weil diese kalorienfrei sind und keine Karies verursachen. Ebenso im Trend liegen nicht nur die klassischen Teesorten, wie Earl Grey, sondern auch Chai-Tees, Mate-Tees und dergleichen. Beiden so unterschiedlichen Produktgruppen ist jedoch ein Problem gemeinsam: synthetische oder pflanzliche Süßstoffe sind zwar frei von den unerwünschten Wirkungen des Zuckers, vermitteln aber doch nicht das gleiche Geschmackserlebnis. Tee, aber auch viele Fruchtsaftzubereitungen, enthalten große Mengen Flavonoide, speziell Catechine sowie ähnliche Flavonoide und andere sekundäre Pflanzenstoffe wie Terpene oder Polyphenole, die zwar als gesundheitsfördernd gelten und daher als Inhaltsstoffe durchaus erwünscht sind, jedoch abhängig von ihrer Menge einen intensiven bitteren, adstringierenden und metallischen Geschmack vermitteln.

Daher besteht im Markt ein intensives Bedürfnis nach Produkten, die in der Lage sind, sowohl die unangenehmen Geschmackseindrücke von Süßstoffen, aber - gegebenenfalls auch beim gemeinsamen Einsatz mit diesen Süßstoffen - die unerwünschten Geschmackserlebnisse von Flavonoiden, Alkaloiden, Terpenen und anderen sekundären Pflanzenstoffen zu maskieren, zu unterdrücken oder zu modulieren, d.h. in einen angenehmen Geschmack umzuwandeln.

Aus dem Stand der Technik sind bereits geschmacksmodulierende Naturstoffe, speziell auch so genannte "Bitterblocker" bekannt. So werden beispielsweise in der DE 10 2012 214560 A1 für diesen Zweck 1,3-Enterodiol-Verbindungen und in EP1258200A2 Flavanone, insbesondere Eriodictylderivate, vorgeschlagen. Aus der EP 2559346 A1 sind Triterpenglykoside vom Oleanen-Typ für den gleichen Einsatzzweck bekannt. Gegenstand der WO 2011 050955 A1 sind Antagonisten und Agonisten für die menschlichen Bittergeschmack-RezeptorenTAS2R40, hTAS2R43, hTAS2R44, hTAS2R46, und hTAS2R47, bei denen es sich ebenfalls um Terpenderivate handelt. Schließlich wird in der WO 2013 072332 A1 über die Verwendung von Hardwickiasäure zur Bekämpfung des Bittergeschmacks berichtet. US2014080842 A1 zeigt am Beispiel von Menthol, dass Dehydroabietinsaure als TRPA1 Antagonist wirksam ist.

Der Einsatz von Naturstoffen, speziell auch von Terpenderivaten zur Bekämpfung, Überdeckung oder Veränderung von unerwünschten Geschmackseigenschaften ist also im Stand der Technik hinreichend beschrieben. Der Nachteil der genannten Verbindungen besteht indes darin, dass sie hohe Einsatzmengen erfordern und insbesondere bezüglich synthetischer Süßstoffe und Flavonoide nur sehr speziell wirksam sind. In den beschriebenen Einsatzmengen besitzen die Stoffe zudem selbst auch wieder einen Eigengeschmack, der das gewünschte Geschmackserlebnis verfälscht und in der Regel verschlechtert. Im Übrigen ist die Wirksamkeit derartiger Wirkstoffe stark von der Matrix, in der sie eingesetzt werden abhängig. Für Hersteller von Nahrungsmitteln ist es daher von großer Wichtigkeit, unter einer großen Zahl von alternativen Wirkstoffen auswählen zu können, um den für den konkreten Anwendungszweck optimalen Stoff zu ermitteln.

Die Aufgabe der vorliegenden Erfindung hat daher darin bestanden, einen Naturstoff zur Verfügung zu stellen, der schon in sehr kleinen Mengen unangenehme Geschmackseindrücke sowohl von Süßstoffen, speziell von Saccharin und Acesulfam K als auch von sekundären Pflanzenstoffen wie anorganischen Salzen, Flavonoiden, Polyphenolen, Alkaloiden und Terpenen, speziell solchen, die in unterschiedlichen Teesorten insbesondere Früchte- und Kräutertee und in Getränken aus Zitrusfrüchten vorhanden sind, überdeckt, neutralisiert oder vorteilhaft verändert.

### BESCHREIBUNG DER ERFINDUNG

Ein erster Gegenstand der Erfindung betrifft Zubereitungen, umfassend oder bestehend aus
(a) Dehydroabietinsäure, einem Dehydroabietinsäure-Salz und/oder einem Dehydroabietinsäure-haltigen Extrakt und
(b1) mindestens einem von Saccharose verschiedenen Süßstoff ausgewählt sind aus der Gruppe, die gebildet wird von Miraculin, Monellin, Thaumatin, Curculin, Brazzein, Magap, Natriumcyclamat, Acesulfam K, Neohesperidin-Dihydrochalcon, Naringin-Dihydrochalcone, Saccharin Natriumsalz, Aspartam, Superaspartam, Neotam, Alitam, Sucralose, Stevioside, Rebaudioside, Lugduname, Carrelame, Sucrononate, Sucrooctate, Monatin, Hernandulcin, Phyllodulcin, Dihydrochalconglykoside, Glycyrrhizinsäure sowie ihre Derivate und Salze, Extrakte von Lakritz (Glycyrrhiza ssp.), Lippia dulcis Extrakte, Momordica ssp. Extrakte, Mogroside sowie deren Gemische, Saccharin, Steviolglykosiden sowie Stevia-Extrakten und/oder
(b2) mindestens einem in Lebensmitteln eingesetzten anorganischen Salz bzw. Mineralstoff ausgewählt sind aus der Gruppe, die gebildet wird von Kalium- und Magnesiumsalzen und deren Gemischen und/oder
(b3) Limonin worin die Zubereitungen die Komponenten (a) in Konzentrationen von 10 bis 250 µM enthalten.

Überraschenderweise wurde gefunden, dass Dehydroabietinsäure das eingangs beschriebene komplexe Anforderungsprofil voll umfänglich erfüllt. Es verbessert sowohl den Geschmackseindruck von künstlichen wie natürlichen Süßstoffen als auch von unterschiedlichen Teesorten und anderen Produkten, bei denen unangenehme, bittere oder adstringierende Geschmackseindrücke durch Flavonoide, das Alkaloid Coffein und das Monoterpen Menthol hervorgerufen werden und dies über eine sehr große Breite unterschiedlicher Spezies. Es handelt sich dabei um ein rein pflanzliches Produkt, das auch noch in sehr geringen Konzentrationen von beispielsweise 25 µM (7,5 mg/l) voll wirksam ist und sich auf diese Weise alternativen Stoffen aus dem Stand der Technik als überlegen erweist.

Unter Geschmackseindrücken, insbesondere unangenehmen Geschmackseindrücken, die es im Sinne der Erfindung zu verbessern gilt, sind insbesondere bittere und adstringierende Geschmacksempfindungen sowie andere Arten von Beigeschmack zu verstehen, die z.B. als Lakritz-ähnlich beschrieben werden. Letztere treten insbesondere im Zusammenhang mit dem Einsatz von Stevia-Extrakten auf.

### DEHYDROABIETINSÄURE

Das Diterpen Dehydroabietinsäure (Komponente (a)) kommt in vielen Nadelbaumgewächsen (u.a. in den Pflanzenfamilien der Pinaceae und Juniperaceae, dabei insbes. in den Gattungen Pinus, Abies, Larix, Juniperus) sowohl in den Nadeln, als auch in Rinde, Wurzel und insbesondere im Harz vor. Es fällt als Abfallprodukt bei der Papierherstellung an und ist im sog. Tallöl enthalten.

Auch aus anderen Pflanzenfamilien wurde Dihydroabietinsäure isoliert (z.B. aus den Gattungen Illicium, Liquidambar, Styrax, Callicarpa, Rosmarinus, Salvia, Commiphora, Boswellia). Besonders häufig kommt Dehydroabietinsäure in Harzen vor (z.B. Liquidambar, Styrax, Boswellia, Commiphora, Colophonium, aber auch in Bernstein).

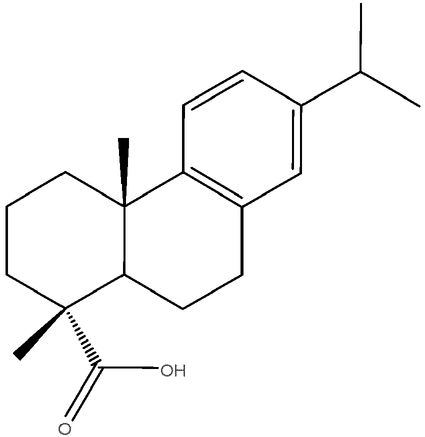

### Strukturformel von Dehydroabietinsäure (CAS 1740-19-8)

Der Gehalt an Dehydroabietinsäure im Kiefernharz beträgt meist unter 10%, kann aber durch eine dem Fachmann als Disproportionierung bekannte Methode (z.B. nach dem von Song et al in JOURNAL OF WOOD CHEMISTRY AND TECHNOLOGY, 5(4), 535-542 (1985) beschriebenen Verfahren) auf einen Gehalt von >50% gesteigert werden.

Es sind beide Enantiomere der Dehydroabietinsäure bekannt (CAS 1740-19-8 und CAS 6980-63-8). Im Sinne der vorliegenden Erfindung sind unter dem Begriff "Dehydroabietinsäure" beide Isomere sowie beliebige Enantiomerengemische zu verstehen.

Obschon auch Extrakte, die Dehydroabietinsäure enthalten, und die handelsübliche technische Dehydroabietinsäure mit einem Reinheitsgrad von etwa 85 % eingesetzt werden kann, ist der Einsatz von hochreinen Produkten mit Dehydroabietinsäuregehalten von mindestens 90 Gew.-%, vorzugsweise mindestens Gew.-95 % und insbesondere etwa 95 bis etwa 99 Gew.-% bevorzugt. Solche hochreinen Produkte sind mit den üblichen Aufarbeitungsverfahren der präparativen organischen Chemie erhältlich, so dass der Fachmann hierzu nicht erfinderisch tätig werden muss. Nachfolgend wird im Beispiel 1 ein entsprechendes Verfahren exemplarisch beschrieben.

Anstelle der Dehydroabietinsäure selbst können auch deren Salze, speziell deren Alkali- und Ammoniumsalze, insbesondere das Natriumsalz eingesetzt werden.

Die Erfindung umfasst auch den Einsatz von Dehydroabietinsäure-haltigen Extrakten, deren Herstellung ebenfalls zum Handwerkszeug des Fachmanns gehört und exemplarisch im nachfolgenden Beispiel 2 beschrieben ist. Vorzugsweise enthalten diese Extrakte mindestens 20 Gew.-%, insbesondere mindestens 30 Gew.-% und besonders bevorzugt etwa 40 bis etwa 60 Gew.-% Dehydroabietinsäure.

### SÜSSSTOFFE

Als Süßstoffe oder süß schmeckende Zusatzstoffe, welche die Gruppe (b1) bilden, kommen zunächst Kohlenhydrate wie z.B. Trehalose, Lactose, Maltose, Melizitose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glycerinaldehyd, oder Maltodextrin in Betracht. Ebenfalls geeignet sind pflanzliche Zubereitungen, die diese Stoffe enthalten, beispielsweise auf Basis von Zuckerrüben (Beta vulgaris ssp., Zuckerfraktionen, Zuckersirup, Melasse), Zuckerrohr (Saccharum officinarum ssp., Melasse, Zuckerrohrsirup), Ahornsirup (Acer ssp.) oder Agaven (Agavendicksaft).

In Betracht kommen auch synthetische, d.h. in der Regel enzymatisch hergestellte Stärke oder Zuckerhydrolysate (Invertzucker, Fructosesirup) sowie natürliche oder synthetische süß schmeckende Substanzen, wie
- Fruchtkonzentrate (z.B. auf Basis von Äpfeln oder Birnen);
- Zuckeralkohole (z.B. Erythritol, Threitol, Arabitol, Ribotol, Xylitol, Sorbitol, Mannitol, Dulcitol, Lactitol);
- Proteine (z.B. Miraculin, Monellin, Thaumatin, Curculin, Brazzein);
- Süßstoffe (z.B. Magap, Natriumcyclamat, Acesulfam K, Neohesperidin Dihydrochalcon, Saccharin Natriumsalz, Aspartam, Superaspartam, Neotam, Alitam, Sucralose, Stevioside, Rebaudioside, Lugduname, Carrelame, Sucrononate, Sucrooctate, Monatin, Phenylodulcin);
- Süß schmeckende Aminosäuren (z.B. Glycin, D-Leucin, D-Threonin, D-Asparagin, D-Phenylalanin, D-Tryptophan, L-Prolin);
- Weitere süß schmeckende niedermolekulare Substanzen, wie z.B. Hernandulcin, Phyllodulcin, Dihydrochalconglykoside, Glycyrrhizin, Glycyrrhizinsäure sowie ihre Derivate und Salze, Rubusoside, Mogroside
- Extrakte süß schmeckender Pflanzen wie Stevia rebaudiana, Glycyrrhiza ssp. (Lakritz), Lippia dulcis, Momordica grosvenori.

Bei den Süßstoffen kann es sich auch um pflanzliche Extrakte handeln, wie nachstehend exemplarisch beschrieben.

**Rebaudioside** gehören zu den Steviosiden, den Hauptbestandteilen der Pflanze *Stevia rebaudiana,* die auch als Süßkraut oder Honigkraut bezeichnet wird. 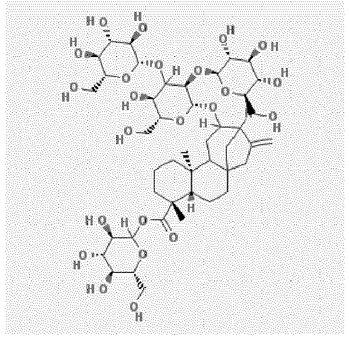 Rebaudiosid

10 % der Trockenmasse der Blätter macht das Diterpenglykoid Steviosid aus, gefolgt von Rebaudiosid A (2 bis 4 Gew.-%) sowie mehr als zehn weiteren Steviolglycosiden, wie etwa dem Dulcosid. Rebaudioside und Stevia-Extrakt sind als Süßungsmitteln inzwischen in den meisten Staaten zugelassen; eine tägliche Aufnahmemenge von bis zu 4 mg Steviosid pro Kilogramm Körpergewicht wird als unbedenklich angesehen. Im Sinne der Erfindung können einzelne Rebaudioside oder die Extrakte der Stevia-Pflanze eingesetzt werden. Besonders bevorzugt ist jedoch die Verwendung von Rebaudiosid A, da dieser Stoff eine geringere Bitterkeit und die höchste Süßkraft aufweist. Die erfindungsgemäßen Stoffgemische können die Komponenten (a) und (b) im Gewichtsverhältnis von etwa 1:99 bis etwa 99:1, vorzugsweise etwa 25:75 bis etwa 75:25 und insbesondere etwa 40:60 bis etwa 60:40 enthalten

Auch die **Dihydrochalcone** stellen Flavonoide dar, wobei insbesondere die beiden Vertreter Naringenin-Dihydrochalcon und Neohesperidin-Dihydrochalcon hervorzuheben sind, die als künstliche Süßungsmittel bekannt sind:

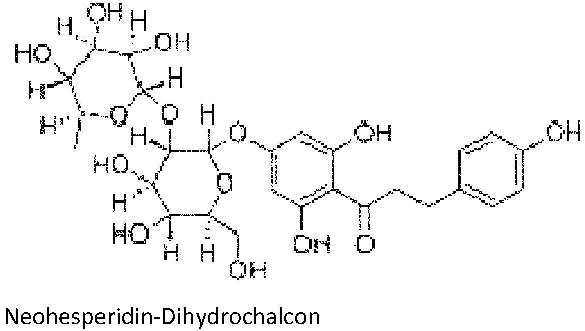

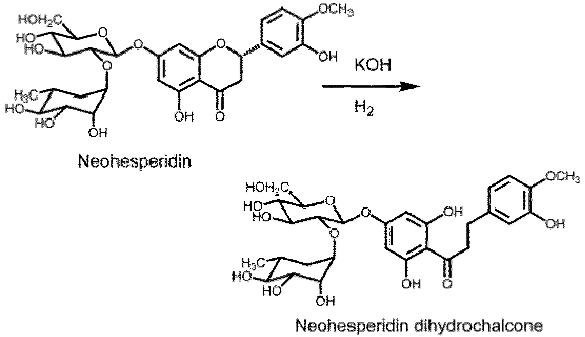

Unter der Bezeichnung **Mogroside** wird eine Gruppe von Glykosiden des Cucurbitans verstanden, die als Bestandteil des natürlichen Süßungsmittels Luo Han Guo bekannt sind. Hervorzuheben ist hier das Mogrosid-V, das 400mal süßer als Zucker ist.

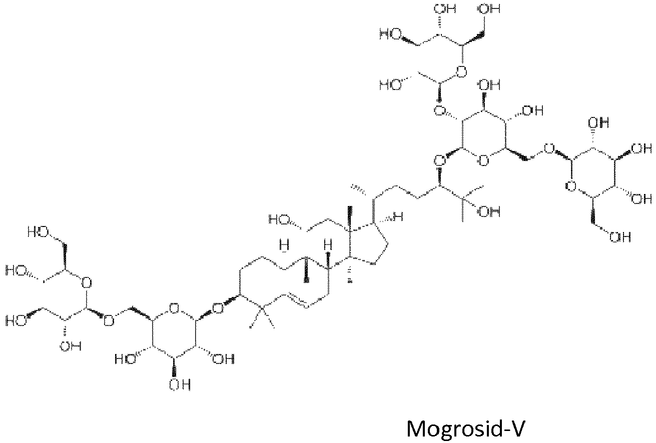

Schließlich kommen als Süßstoffe auch Extrakte der Pflanzen in Betracht, die ausgewählt sind aus der Gruppe, die gebildet wird von *Rubus allegheniensis, Rubus arcticu, Rubus strigosus, Rubus armeniacus, Rubus caesius, Rubus chamaemorus, Rubus corylifolius agg., Rubus fruticosus agg., Rubus geoides, Rubus glaucus, Rubus gunnianus, Rubus idaeus, Rubus illecebrosus, Rubus laciniatus, Rubus leucodermis, Rubus loganobaccus, Rubus loxensis, Rubus nepalensis, Rubus nessensis, Rubus nivalis, Rubus odoratus, Rubus pentalobus, Rubus phoenicolasius, Rubus saxatilis, Rubus setchuenensis, Rubus spectabilis* und *Rubus ulmifolius* sowie deren Gemischen. Hierbei handelt es sich im Wesentlichen um Extrakte von unterschiedlichen Brombeer- und Himbeerarten, die einen Gehalt an Rubosiden aufweisen. Bevorzugt sind Extrakte von *Rubus suavissimus.*

Ein weiterer Wirkstoff in dieser Gruppe ist die **Glycyrrhizinsäure** oder ein entsprechendes Salz oder einen Extrakt, der diesen Stoff enthält.

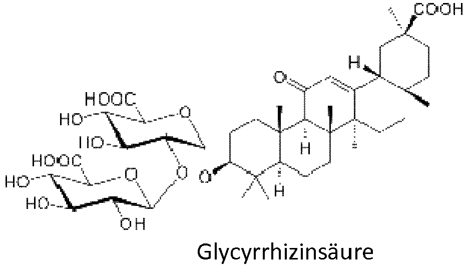

Im Sinne der Erfindung ist es möglich, die Säure selbst, ihre Salze - beispielsweise Natrium-, Kalium- oder Ammoniumsalz - oder die Extrakte der Pflanze *Glycyrrhiza glabra* einzusetzen. Besonders bevorzugt ist das Monoammoniumglycyrrhizat.

Die bevorzugten Süßstoffe, deren geschmackliche Wahrnehmung es zu verbessern gilt, sind ausgewählt sind aus der Gruppe, die gebildet wird von Saccharin, Acesulfam K, Steviolglykosiden, insbesondere Rebaudiosid A, sowie Stevia-Extrakten.

### FLAVONOIDE

Bei den Flavonoiden, handelt es sich in erster Linie um Catechine, die in unterschiedlichen Mengen und Zusammensetzungen vor allem in Tee enthalten sind und zu den stärksten Bitterstoffen zählen. Die wichtigsten Vertreter sind hier: 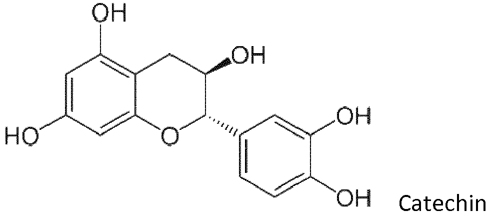 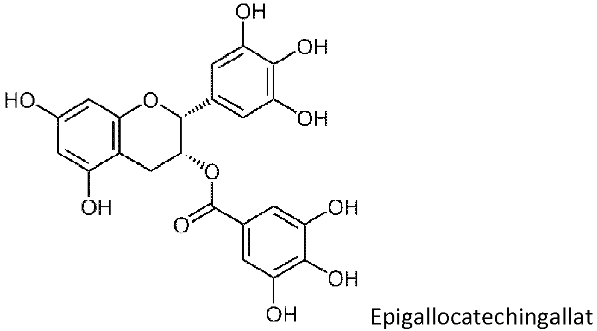 sowie Epicatechin und Gallocatechin. Neben den Flavonoiden und ihren Oligomeren und Oxidationsprodukten trägt auch das Alkaloid Coffein zum bitteren Geschmack von Grünem und Schwarzen Tee bei. In Getränken aus Zitrusfrüchten ist der bittere oder adstringierende Geschmack vor allem auf polymethoxylierte Flavone wie Sinensetin und Nobiletin, glykosylierte Flavonoide, z.B. Naringin und auf Terpene vom Typ der Limonoide, z.B. Limonin, zurückzuführen.

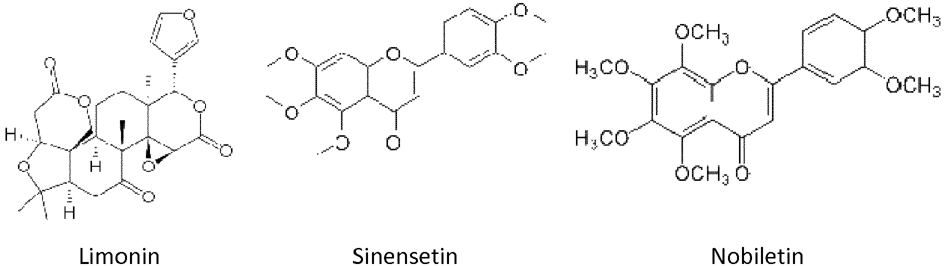

### MINERALSTOFFE

Magnesium ist ein wichtiger Mineralstoff. Unterversorgung mit Magnesium wird häufig nicht erkannt, kann aber als Nebenerscheinung bei vielen Krankheiten (z.B. Diabetes, Bluthochdruck) auftreten. Einen erhöhten Bedarf an Magnesium haben häufig ältere Menschen und Schwangere. Ein Mehrbedarf an Magnesium kann durch Mineralstoffpräparate oder durch Magnesiumzusatz zu Nahrungsmitteln, z.B. zu speziell für ältere Menschen hergestelltem Brot, ausgeglichen werden. Magnesium kann in Form unterschiedlicher Salze genommen werden, z.B. als Magnesiumchlorid oder Magnesiumsulfat. Magnesiumhaltige Salze, insbes. das auch als Bittersalz bezeichnete Magnesiumsulfat, schmecken bitter.

Kalium wird ebenfalls in Lebensmitteln zugesetzt, üblicherweise jedoch nicht, um Kaliummangel auszugleichen sondern als Salzersatz, um den Natriumgehalt in Lebensmitteln bei Erhalt des Salzgeschmacks zu reduzieren. Da Kaliumchlorid einen bitteren Beigeschmack hat, kann nur ein Teil des Natriums durch Kalium ersetzt werden. Zur Reduktion des Bittergeschmacks von Kaliumchlorid können zum Beispiel Ascorbinsäure, Fumarsäure und Citronensäure oder unterschiedliche Zucker (Lactose, Dextrose) oder Hefeextrakt zugesetzt werden (s. DE3035518C2). Diese Lösungen eignen sich aber nur für wenige Lebensmittel, da sie geschmacklich nicht neutral sind oder durch Zusatz von Zucker zu einer vermehrten Kalorienaufnahme führen, die in kochsalzreduzierten, und damit gesünderen Lebensmitteln nicht erwünscht ist.

### ZUBEREITUNGEN

Zubereitungen der vorliegenden Erfindung können die Komponenten (a) bezogen auf Komponente (b) in Konzentrationen von etwa 20 bis etwa 100 µM enthalten.

Eine Zubereitungsform, die für den Handel bestimmt und besonders geeignet ist, beispielsweise in Form einer Süßstofftablette, enthält typisch 1 bis 40 mg Süßstoff(e), z.B. Saccharin oder Acesulfam K, und 1 bis 30 mg Dehydroabietinsäure, bezogen auf ein Gesamtgewicht der Tablette von 60 mg wobei die Differenzmenge Tablettierungshilfsmittel, insbesondere Binder ausmachen. Eine solche Tablette entspricht der Süßkraft von einem Stück Würfelzucker und ist vollständig ausreichend um beispielsweise eine gewöhnliche Tasse Tee zu süßen und dabei den mit der Zugabe des Süßstoffs verbundenen unangenehmen Geschmack vollständig zu überdecken.

### NAHRUNGSMITTEL

Ein weiterer Gegenstand der vorliegenden Erfindung betrifft Nahrungsmittel, die die erfindungsgemäßen Zubereitungen enthalten, wobei die Dehydroabietinsäure oder ein Dehydroabietinsäure-Salz in Konzentrationen von 1 bis 250 µM enthalten ist. Dabei ist es möglich die Komponenten einzeln oder gemeinsam zuzugeben, wobei die Zugabe der Mischung bevorzugt ist. Im Hinblick auf die Geschmacksverbesserung bei den Flavonoiden, kann die Komponente (a) alleine oder als Mischung von (a) und (b) eingesetzt werden.

Beispiel für Nahrungsmittel umfassen grundsätzlich Backwaren, beispielsweise Brot, Trockenkekse, Kuchen, sonstiges Gebäck, Süßwaren (beispielsweise Schokoladen, Schokoladenriegelprodukte, sonstige Riegelprodukte, Fruchtgummi, Hart- und Weichkaramellen, Kaugummi), alkoholische oder nicht-alkoholische Getränke (beispielsweise Kaffee, Tee, Eistee, Wein, weinhaltige Getränke, Bier, bierhaltige Getränke, Liköre, Schnäpse, Weinbrände, (karbonisierte) fruchthaltige Limonaden, (karbonisierte) isotonische Getränke, (karbonisierte) Erfrischungsgetränke, Nektare, Schorlen, Obst- und Gemüsesäfte, Frucht- oder Gemüsesaftzubereitungen, Instantgetränke (beispielsweise Instant-Kakao-Getränke, Instant-Tee-Getränke, Instant-Kaffeegetränke, Instant-Fruchtgetränke)), Fleischprodukte (beispielsweise Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (beispielsweise Frühstückscerealien, Müsliriegel, vorgegarte Fertigreis-Produkte), Milchprodukte (beispielsweise Milchgetränke, Buttermilchgetränke, Milcheis, Joghurt, Kefir, Frischkäse, Weichkäse, Hartkäse, Trockenmilchpulver, Molke, Molkegetränke, Butter, Buttermilch, teilweise oder ganz hydrolysierte Milchprotein-haltige Produkte), Produkte aus Sojaprotein oder anderen Sojabohnen-Fraktionen (beispielsweise Sojamilch und daraus gefertigte Produkte, Fruchtgetränke mit Sojaprotein, Sojalecithin-haltige Zubereitungen, fermentierte Produkte wie Tofu oder Tempe oder daraus gefertigte Produkte), Produkte aus anderen pflanzlichen Proteinquellen, beispielsweise Haferprotein-Getränke, Fruchtzubereitungen (beispielsweise Konfitüren, Fruchteis, Fruchtsoßen, Fruchtfüllungen), Gemüsezubereitungen (beispielsweise Ketchup, Soßen, Trockengemüse, Tiefkühlgemüse, vorgegarte Gemüse, eingekochte Gemüse), Knabberartikel (beispielsweise gebackene oder frittierte Kartoffelchips oder Kartoffelteigprodukte, Extrudate auf Mais- oder Erdnussbasis), Produkte auf Fett- und Ölbasis oder Emulsionen derselben (beispielsweise Mayonnaise, Remoulade, Dressings), sonstige Fertiggerichte und Suppen (beispielsweise Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden.

Vorzugsweise handelt es sich jedoch um Erfrischungsgetränke oder Heißgetränke oder Instantgetränke, wie beispielsweise Tees oder Fruchtsäfte.

### PHARMAZEUTISCHE ZUBEREITUNGEN

Das eingangs geschilderte Geschmacksproblem tritt nicht nur bei Nahrungsmitteln, denen man zuckerfremde Süßstoffe zusetzt auf, sondern auch bei Pharmaprodukten, bei denen der häufig abstoßende Geschmack des Wirkstoffs besonders sorgfältig überdeckt werden muss. Daher betrifft ein weiterer Gegenstand der Erfindung auch pharmazeutische Zubereitungen, die die erfindungsgemäßen Zubereitungen enthalten, wobei die Dehydroabietinsäure oder ein Dehydroabietinsäure-Salz in Konzentrationen von 1 bis 250 µM enthalten ist. Auch hier ist die Zugabe der Komponente (a) und (b) als Mischung bevorzugt. Vorzugsweise handelt es sich bei den pharmazeutischen Zubereitungen um Flüssigprodukte, insbesondere um Hustensaft, fiebersenkende Mittel oder Antibiotika. Insbesondere bei Kindern werden flüssige Arzneiformen eingesetzt, bei denen es technologisch nicht möglich ist, den bitteren Geschmack des Wirkstoffes zu überdecken wie bei der Applikation von Kapseln, Tabletten oder anderen festen Arzneiformen.

Sowohl die Nahrungsmittel als auch die pharmazeutischen Zubereitungen können die Dehydroabietinsäure oder ein Dehydroabietinsäure-Salz in Konzentrationen von vorzugsweiseetwa 20 bis etwa 100 µM enthalten.

### GEWERBLICHE ANWENDBARKEIT

Ein weiterer Gegenstand der Erfindung betrifft ein Verfahren zur Maskierung unangenehmer, insbesondere bitterer, adstringierender und/oder Lakritz-artiger Geschmackseindrücke von Miraculin, Monellin, Thaumatin, Curculin, Brazzein, Magap, Natriumcyclamat, Acesulfam K, Neohesperidin-Dihydrochalcon, Naringin-Dihydrochalcone, Saccharin Natriumsalz, Aspartam, Superaspartam, Neotam, Alitam, Sucralose, Stevioside, Rebaudioside, Lugduname, Carrelame, Sucrononate, Sucrooctate, Monatin, Hernandulcin, Phyllodulcin, Dihydrochalconglykoside, Glycyrrhizinsäure sowie ihre Derivate und Salze, Extrakte von Lakritz (Glycyrrhiza ssp.), Lippia dulcis Extrakte, Momordica ssp. Extrakte, Mogroside sowie deren Gemische, Saccharin, Steviolglykosiden sowie Stevia-Extrakten und/ oder Kaliumsalzen sowie Nahrungsmitteln oder pharmazeutischen Zubereitungen, die diese Stoffe enthalten, dadurch gekennzeichnet, dass man diesen eine Menge an Dehydroabietinsäure, einem Dehydroabietinsäure-Salz und/oder einem Dehydroabietinsäure-haltigen Extrakt zusetzt.

Vorzugsweise wird die Dehydroabietinsäure, ein Dehydroabietinsäure-Salz und/oder ein Dehydroabietinsäure-haltiger Extrakt in Mengen zugesetzt, so dass die Konzentration der Dehydroabietinsäure im Produkt etwa 1 bis etwa 500 µM, vorzugsweise etwa 10 bis etwa 250 µM, insbesondere etwa 20 bis etwa 150 µM und insbesondere etwa 50 bis etwa 100 µM beträgt.

Ebenfalls beansprucht wird die Verwendung von Dehydroabietinsäure, einem Dehydroabietinsäure-Salz und/oder einem Dehydroabietinsäure-haltigen Extrakt zur Maskierung unangenehmer, insbesondere bitterer, adstringierender und/oder Lakritz-artiger Geschmackseindrücke von Miraculin, Monellin, Thaumatin, Curculin, Brazzein, Magap, Natriumcyclamat, Acesulfam K, Neohesperidin-Dihydrochalcon, Naringin-Dihydrochalcone, Saccharin Natriumsalz, Aspartam, Superaspartam, Neotam, Alitam, Sucralose, Stevioside, Rebaudioside, Lugduname, Carrelame, Sucrononate, Sucrooctate, Monatin, Hernandulcin, Phyllodulcin, Dihydrochalconglykoside, Glycyrrhizinsäure sowie ihre Derivate und Salze, Extrakte von Lakritz (Glycyrrhiza ssp.), Lippia dulcis Extrakte, Momordica ssp. Extrakte, Mogroside sowie deren Gemische, Saccharin, Steviolglykosiden sowie Stevia-Extrakten und/oder Kaliumsalzen sowie Nahrungsmitteln oder pharmazeutischen Zubereitungen, die diese Stoffe enthalten. Bevorzugt setzt man diesen Stoffen und Zubereitungen die Dehydroabietinsäure, ein Dehydroabietinsäure-Salz und/oder einen Dehydroabietinsäurehaltigen Extrakt so zu, dass die Konzentration der Dehydroabietinsäure im Produkt etwa 1 bis etwa 500 µM, vorzugsweise etwa 10 bis etwa 250 µM, insbesondere etwa 20 bis etwa 100 µM beträgt.

### BEISPIELE

### BEISPIEL 1

### Reinigung von Dehydroabietinsäure

2g handelsübliche Dehydroabietinsäure (CAS 1740-19-8, bezogen von Interchim, 211 bis AVENUE KENNEDY, BP 1140, 03103 MONTLUCON CEDEX, Frankreich) mit einem Gehalt von ca. 85% wurde durch präparative HPLC-Chromatographie wie folgt gereinigt.

| | |
|---|---|
| Trennungsnummer: | H-2074-B |
| Stationäre Phase: | LichrospherSelect B, 10 µm, 250 x 50 mm |
| Mobile Phase A: | 5mMol Ammoniumformiatpuffer, mit Ameisensäure auf pH 3,0 eingestellt |
| Mobile Phase B: | Methanol - Acetonitril 1:1 (v/v) mit 5 mMol Ammoniumformiat |
| Gradient: | 62% auf 81% B in 57 min |
| Flussrate: | 80 ml/min |
| Detektion: | ELSD |

Produkthaltige Fraktionen wurden vereinigt, das Lösemittel im Vakuum abgedampft und die isolierte Dehydroabietinsäure über H-NMR-Spektroskopie und LCMS analytisch charakterisiert. Die Identität der isolierten Dehydroabietinsäure wird durch das NMR und die Molmasse bestätigt, die Reinheit lag bei > 98%.

Das LCMS-Verfahren zur analytischen Charakterisierung der isolierten Substanz ist in der nachfolgenden **Tabelle 1** zusammengefasst:

**Tabelle 1**

| LCMS Verfahren | | | |
|---|---|---|---|
| **HPLC System** | PE Series 200 | | |
| **MS System** | Applied Biosystems API 150 | | |
| **Data System** | Analyst 1.3 | | |
| **Stationäre Phase** | Merck Select B 250x4 mm, 5 µm | | |
| **Flussrate** | 1 ml/min | | |
| **Detektion** | (+/(-)-ESI, Fast-Switching-Mode | | |
| | ELSD (Sedex 75) | | |
| | UV (Merck, 254 nm) | | |
| **Probenkonzentration** | 10 mg/ml in DMSO | | |
| **Injektionsvolumen** | 30 µl | | |
| **Mobile Phase:** | A: 5 mM Ammoniumformiat and 0.1 % Ameisensäure | | |
| | B: Acetonitril/methanol = 1:1, 5 mM Ammoniumformiat und 0.1 % Ameisensäure (pH 3) | | |
| **Gradient** | Time [min] | % A | % B |
| | 00.0 | 85 | 15 |
| | 30.0 | 0 | 100 |
| | 35.0 | 0 | 100 |

### BEISPIEL 2

### Herstellung eines dehydroabietinsäurehaltigen Extraktes

10g Handelsübliches Colophonium-Harz (bezogen von der Alfred Galke GmbH, Am Bahnhof 1, 37539 Bad Grund, Bestellnummer 36004) wird mit einem Gemisch aus MTB-Ether und Methanol (1:1 v/v) extrahiert und das Lösemittel im Vakuum entfernt. Der Extrakt enthält ca. 25% Dehydroabietinsäure.

### BEISPIEL 3

### In vitro Assay

Um natürliche Substanzen mittels eines zellbasierten Testsystems zu finden, welche die Bitterkeit von Bitterstoffen reduzieren, wurden humane Bittergeschmackszellen etabliert und die Aktivierung dieser Zellen quantifiziert. Zur Quantifizierung der Aktivierung von Bittergeschmackszellen, wurde die Änderung des intrazellulären Kalziumspiegels gemessen.

### Etablierung des zellbasierten Messsystems

Um Substanzen mit inhibitorischer Aktivität zu finden, wurde ein hochdurchsatzfähiges, zellbasiertes *in-vitro* Messsystem etabliert. Hierzu wurde eine humane Bittergeschmackszelllinie genutzt, die in Anlehnung an Hochheimer, A., Krohn, M., Rudert, K., Riedel, K., Becker, S., Thirion, C. and Zinke, H. "Endogenous gustatory responses and gene expression profile of stably proliferating human taste cells isolated from fungiform papillae" Chem Senses, 39, 359-377 (2014**),** für diese Anwendung hergestellt wurde.

### Quantifizierung des intrazellulären Kalziumspiegels im zellbasierten Messsystem

Für ein Screening nach Antagonisten im Hochdurchsatzformat wurde eine humane Bittergeschmackszelllinie verwendet, welche endogen über Bitterrezeptoren verfügt. Die Modulierung des intrazellulären Kalziumspiegels der Bittergeschmackszellen wurde mittels des Kalzium-sensitiven Fluoreszenzfarbstoffs Fluo-4 AM in einem Mikroplattenlesegerät quantifiziert. Die Aktivierung der Bittergeschmackszellen durch einen Agonisten führt zu einem Anstieg des intrazellulären Kalziumspiegels. Die Menge des mobilisierten Kalziums kann durch die Fluoreszenz quantifiziert werden, welche proportional zur Kalziumkonzentration ansteigt. Im Falle einer Inhibition durch einen Antagonisten, ist die Menge des mobilisierten Kalziums und somit die Änderung der Fluoreszenz, die durch einen Agonisten induziert wird, signifikant geringer.

*Durchführung:* Humane Bittergeschmackszellen wurden in Zellkulturmedium, in einer Wasserdampf-gesättigten Atmosphäre bei 37°C und 5% CO2 kultiviert. Die Zellen wurden mit einer Dichte von 20.000 Zellen pro Loch in eine 96-Lochplatte ausgesät. Die Zellen wurden 24 Stunden nach der Aussaat mit 4 µM des Kalzium-sensitiven Fluoreszenzfarbstoffs Fluo-4 AM in HBSS (*Hank's* Balanced Salt Solution)-Puffer gefärbt und die Mobilisierung von Kalzium in lebenden Zellen im Mikroplattenlesegerät (FlexStation®System, Molecular Devices) gemessen.

*Antagonist-Screening:* Humane Bittergeschmackszellen wurden parallel mit 1-5 mM des Bitterstoffs Saccharin (Agonist) und 10 µM der potentiellen Antagonisten stimuliert. Die Mobilisierung des Kalziums wurde im Mikroplattenlesegerät bei 37 °C gemessen.

*Auswertung:* Die Mobilisierung des Kalziums wurde als Änderung der Fluoreszenz (dF) nach Substanzzugabe unter Berücksichtigung der Basalfluoreszenz (F0) als dF/F0 quantifiziert. Die relativen Fluoreszenzeinheiten (dF/F0) gemessen nach Stimulation mit Agonist und Antagonist wurden mit der alleinigen Stimulation durch den Agonist (Positivkontrolle) verglichen. So wurden aktive Substanzen, die den Kalziumspiegel senken identifiziert. Potentielle Antagonisten wurden wiederholt auf ihre Aktivität getestet. Die Verarbeitung und Analyse der Daten erfolgte mit einem Programm des Mikroplattenlesegerätes (SoftMax®Pro Software, Molecular Devices).

### Bestimmung der Dosis-Wirk-Beziehung von Antagonisten

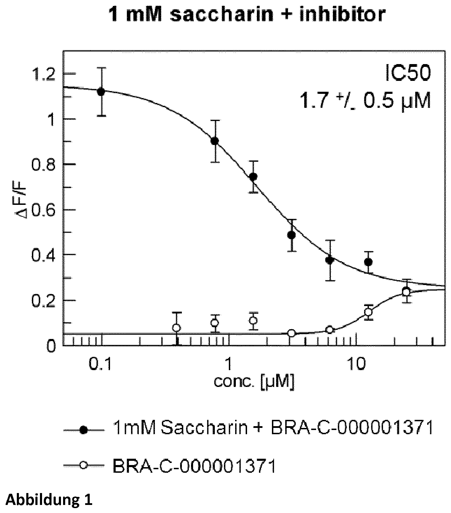

Zur Bestimmung der mittleren inhibitorischen Konzentration (IC50) des Antagonisten wurden humane Bittergeschmackszellen mit 0,1-25 µM der Substanz und unter Gegenwart des Agonisten stimuliert. Eine repräsentative Dosis-Wirk-Beziehung ist in **Abbildung 1** dargestellt. Angegeben ist die Dosis-Wirkungs-Beziehung von Dehydroabietinsäure (BRA-C-00001371) in humanen Geschmackszellen bei Stimulierung durch 1 mM Saccharin (fluorometrische Detektion) IC50 1,7 +/- 0,5 µM

### BEISPIEL 4

### Eigengeschmack hochreiner Dehydroabietinsäure sowie Dehydroabietinsäure-haltiger Extrakte

Die sensorische Beurteilung der Proben wurde von einem Team aus fünf erfahrenen Prüfern durchgeführt. Die Sensorischen Prüfungen erfolgten deskriptiv, randomisiert, verblindet und mittels einer "sip and spit" Methode. Dabei wird die Probe für einige Sekunden im Mund bewegt, um sie geschmacklich zu bewerten, und nicht geschluckt sondern ausgespuckt. Die Ergebnisse sind der folgenden **Tabelle 2a** zu entnehmen:

**Tabelle 2a**

| Verkostung Dehydroabietinsäure, hochrein | | |
|---|---|---|
| **Testmethode** | deskriptive Prüfung, "sip and spit"-Methode, Proben verblindet und randomisiert, pH neutral | |
| **Testprobe** | gereinigte Dehydroabietinsäure, Reinheit > 98% | |
| **Panelist** | 3-5 erfahrene Testpersonen | |
| **Probenvorbereitung** | drei verschiedene Konzentrationen (10 µM, 50 µM und 100 µM) in 0,5%igem Ethanol gelöst, Vergleichsprobe 0,5% Ethanol in Wasser | |
| **Bewertung** | 10 µM | neutral |
| | 50 µM | adstringierend, trocken, leichter metallischer Beigeschmack |
| | 100 µM | Leicht kühlendes Gefühl auf der Zunge, adstringierend, trocken, leicht bitter, nicht süß, geringfügig den Geschmack von Ethanol maskierend |

**Tabelle 2b**

| Verkostung Dehydroabietinsäure-haltige Extrakte | | | | |
|---|---|---|---|---|
| **Testmethode** | deskriptive Prüfung, "sip and spit"-Methode, Proben verblindet und randomisiert, pH neutral | | | |
| **Testprobe** | 1) gereinigte Dehydroabietinsäure, Reinheit > 98% | | | |
| | 2) handelsübliche Dehydroabietinsäure, Reinheit 85 % (HPLC) | | | |
| | 3) Extrakt mit ca. 25 % Dehydroabietinsäure | | | |
| **Panelist** | 3-5 erfahrene Testpersonen | | | |
| **Probenvorbereitung** | drei verschiedene Konzentrationen (in 0,5%igem Ethanol gelöst), Vergleichsprobe 0,5% Ethanol in Wasser, Endkonzentration des Ethanols unter 0,5% | | | |
| **Bewertung** | Konz. | Probe 1 | Probe 2 | Probe 3 |
| | 3 mg/l | neutral | neutral | neutral |
| | 7,5 mg/l | neutral | neutral | schwach bitter |
| | 30 mg/l | leicht bitter | leicht bitter | bitterer als Probe 1 und 2, harzig |

### BEISPIEL 5

### Geschmack der Kombination von Dehydroabietinsäure mit Saccharin

Die sensorische Beurteilung der Proben wurde von einem Team aus fünf erfahrenen Prüfern durchgeführt. Die Ergebnisse sind der folgenden **Tabelle 3** zu entnehmen:

**Tabelle 3**

| Verkostung Dehydroabieteinsäure, hochrein + Saccharin | | |
|---|---|---|
| **Testmethode** | deskriptive und diskriminierende Prüfung, "sip and spit"-Methode, Proben verblindet und randomisiert, pH neutral | |
| **Testprobe** | gereinigte Dehydroabietinsäure, Reinheit > 98% | |
| **Panelist** | 3-5 erfahrene Testpersonen, trainiert auf den Beigeschmack von Saccharin und Acesulfam K | |
| **Probenvorbereitung** | drei verschiedene Konzentrationen Dehydroabietinsäure (10 µM, 25 µM und 50 µM) mit 80 ppm Saccharin (entspricht 3% Saccharose-Äquivalent) in 0,5%igem Ethanol gelöst, 80 ppm Saccharin Vergleichsprobe 0,5% Ethanol in Wasser, Endkonzentration des Ethanols unter 0,5% | |
| **Bewertung** | 10 µM | Leicht bitterer Nachgeschmack |
| | 25 µM | Weniger bitter mit sehr klarem Geschmacksprofil, weniger trocken |
| | 50 µM | Weniger bitter, deutlich geringerer Nachgeschmack wie bei 10 µM |

### BEISPIEL 6

### Geschmack der Kombination von Dehydroabietinsäure mit Acesulfam K

Die sensorische Beurteilung der Proben wurde von einem Team aus fünf erfahrenen Prüfern durchgeführt. Die Ergebnisse sind der folgenden **Tabelle 4** zu entnehmen:

**Tabelle 4**

| Verkostung Dehydroabieteinsäure, hochrein + Acesulfam K | | |
|---|---|---|
| **Testmethode** | deskriptive und diskriminierende Prüfung, "sip and spit"-Methode, Proben verblindet und randomisiert, pH neutral | |
| **Testprobe** | gereinigte Dehydroabietinsäure, Reinheit > 98% | |
| **Panelist** | 3-5 erfahrene Testpersonen, trainiert auf den Beigeschmack von Saccharin und Acesulfam K | |
| **Probenvorbereitung** | drei verschiedene Konzentrationen Dehydroabietinsäure (10 µM, 25 µM und 50 µM) mit 150 ppm Acesulfam-K (entspricht 3% Saccharose-Äquivalent) in 0,5%igem Ethanol gelöst, 150 ppm Acesulfam-K Vergleichsprobe 0,5% Ethanol in Wasser, Endkonzentration des Ethanols unter 0,5% | |
| **Bewertung** | 10 µM | Sehr runder Geschmackseindruck, deutlich weniger bitter, maskierend bezüglich des adstringierenden und metallischen Beigeschmacks |
| | 25 µM | Keinerlei bitterer Beigeschmack mehr vorhanden |
| | 50 µM | Klarer Geschmackseindruck, verzögert den Anstieg eines bitteren und sauren Beigeschmacks. |

### VERGLEICHSBEISPIEL 7

### Geschmacksmodulierung von Schwarzem Tee

Die sensorische Beurteilung der Proben wurde von einem Team aus fünf erfahrenen Prüfern durchgeführt. Die Einzelheiten sind den folgenden **Tabelle 5** zu entnehmen:

**Tabelle 5**

| Verkostung Schwarzer Tee | | |
|---|---|---|
| **Testmethode** | deskriptive und diskriminierende Prüfung, "sip and spit"-Methode, Proben verblindet und randomisiert, pH neutral | |
| **Testprobe** | gereinigte Dehydroabietinsäure, Reinheit > 98% | |
| **Panelist** | 5 erfahrene Testpersonen | |
| **Probenvorbereitung** | zwei verschiedene Konzentrationen Dehydroabietinsäure (25 µM und 50 µM) gelöst in Ethanol, 1 Teebeutel Earl Grey, 10min ziehen lassen in 100ml, Zugabe der Stammlösung bei ca. 20°C, Endkonzentration des Ethanols unter 0,5%, Vergleichsprobe 0,5% Ethanol in Tee | |
| **Bewertung** | 25 µM | etwas weniger bitter als der Vergleich |
| | 50 µM | Deutlich weniger bitter, etwas weniger adstringierend als der Vergleich |

### VERGLEICHSBEISPIEL 8

### Geschmacksmodulierung von Grünem Tee

Die sensorische Beurteilung der Proben wurde von einem Team aus fünf erfahrenen Prüfern durchgeführt. Die Einzelheiten sind den folgenden **Tabelle 6** zu entnehmen:

**Tabelle 6**

| Verkostung Grüner Tee | | |
|---|---|---|
| **Testmethode** | deskriptive und diskriminierende Prüfung, "sip and spit"-Methode, Proben verblindet und randomisiert, pH neutral | |
| **Testprobe** | gereinigte Dehydroabietinsäure, Reinheit > 98% | |
| **Panelist** | 5 erfahrene Testpersonen | |
| **Probenvorbereitung** | Probenvorbereitung: zwei verschiedene Konzentrationen Dehydroabietinsäure (25 µM und 50 µM) gelöst in Ethanol, 3g Grüner Tee, 5min ziehen lassen in 250ml Wasser, Zugabe der Stammlösung bei ca. 20°C, Endkonzentration des Ethanols unter 0,5%, Vergleichsprobe 0,5% Ethanol in Tee | |
| **Bewertung** | 25 µM | Leicht bitterer Geschmack, der rasch abklingt |
| | 50 µM | Wie bei 25 µM, aber weniger adstringierend |

### BEISPIEL 9

### Geschmacksmodulierung von Grapefruitsaft

Die sensorische Beurteilung der Proben wurde von einem Team aus fünf erfahrenen Prüfern durchgeführt. Die Einzelheiten sind den folgenden **Tabelle 7** zu entnehmen:

**Tabelle 7**

| Verkostung Grapefruitsaft | | |
|---|---|---|
| **Testmethode** | deskriptive und diskriminierende Prüfung, "sip and spit"-Methode, Proben verblindet und randomisiert, pH neutral | |
| **Testprobe** | gereinigte Dehydroabietinsäure, Reinheit > 98% | |
| **Panelist** | 5 erfahrene Testpersonen | |
| **Probenvorbereitung** | zwei verschiedene Konzentrationen Dehydroabietinsäure (25µM und 50 µM) gelöst in Ethanol, Grapefruitsaft (Einzelhandel, aus Konzentrat), Zugabe der Stammlösung bei ca. 20°C, Endkonzentration des zugegebenen Ethanols unter 0,5%, pH der Testprobe 3,56, Vergleichsprobe 0,5% Ethanol in Grapefruitsaft | |
| Bewertung | 25 µM | Deutlich weniger bitter als der Vergleich, deutlich aromatischer |
| | 50 µM | Deutlich weniger bitter als der Vergleich, keine negative Beeinflussung des Fruchtgeschmacks |

Versuche mit Grapefruit-Direktsaft zeigten ebenfalls eine deutliche Reduktion des bitteren Geschmacks ohne negative Beeinflussung des Fruchtaromas und der erfrischenden Säure.

### BEISPIEL 10

### Geschmacksmodulierung einer Mineralsalzlösung

Die sensorische Beurteilung der Proben wurde von einem Team aus fünf erfahrenen Prüfern durchgeführt. Die Einzelheiten sind den folgenden **Tabelle 8** zu entnehmen:

**Tabelle 8**

| Verkostung Mineralsalzlösung | | |
|---|---|---|
| **Testmethode** | deskriptive und diskriminierende Prüfung, "sip and spit"-Methode, Proben verblindet und randomisiert, pH neutral | |
| **Testprobe** | gereinigte Dehydroabietinsäure, Reinheit > 98% | |
| **Panelist** | 5 erfahrene Testpersonen | |
| **Probenvorbereitung** | zwei verschiedene Konzentrationen Dehydroabietinsäure (25 µM und 50 µM) gelöst in Ethanol, 2g MgCl₂ (Lohmann) in 100 ml Wasser gelöst, Zugabe der Stammlösung bei ca. 20°C, Endkonzentration des Ethanols unter 0,5%, Vergleichsprobe: Mineralsalzlösung mit 0,5% Ethanol | |
| Bewertung | 25 µM | Kaum Unterschied zum Vergleich |
| | 50 µM | Wesentlich weniger bitter als der Vergleich |

### BEISPIEL 11

### Geschmacksmodulierung einer Kaliumchlorid-haltigen Lösung

Die sensorische Beurteilung der Proben wurde von einem Team aus fünf erfahrenen Prüfern durchgeführt. Die Einzelheiten sind den folgenden **Tabelle 9** zu entnehmen:

**Tabelle 9**

| Verkostung Kaliumchloridlösung | | |
|---|---|---|
| **Testmethode** | deskriptive und diskriminierende Prüfung, "sip and spit"-Methode, Proben verblindet und randomisiert, pH neutral und 3,2, Vergleichsprobe: 0,5% Ethanol in Kaliumchloridlösung bei entsprechendem pH-Wert | |
| **Testpro be** | gereinigte Dehydroabietinsäure, Reinheit > 98% | |
| **Panelist** | 5 erfahrene Testpersonen | |
| **Proben vorbereitung** | zwei verschiedene Konzentrationen Dehydroabietinsäure (25 µM und 50 µM) gelöst in Ethanol, 1500 mg KCl in 100 ml Wasser gelöst, pH Wert wurde durch Zitronensäure eingestellt, Zugabe der Stammlösung bei ca. 20°C, Endkonzentration des Ethanols unter 0,5% | |
| Bewertung | 25 µM pH neutral | Kaum Unterschied zum Vergleich (Bitterkeit und Salzigkeit) |
| | 25 µM pH 3,2 | Kaum Unterschied zum Vergleich (Bitterkeit) |
| | | Salziger als Vergleich |
| | | Säurebetonter als Vergleich |
| | 50 µM pH neutral | Weniger bitter als der Vergleich |

### BEISPIEL 12

### Geschmacksmodulierung einer Stevia-Rebaudiosid A-Lösung

Die sensorische Beurteilung der Proben wurde von einem Team aus fünf erfahrenen Prüfern durchgeführt. Die Einzelheiten sind den folgenden **Tabelle 10** zu entnehmen:

**Tabelle 10**

| Verkostung Stevia-RebA-Lösung | | |
|---|---|---|
| **Testmethode** | deskriptive und diskriminierende Prüfung, "sip and spit"-Methode, Proben verblindet und randomisiert, pH 3,2 und pH neutral | |
| **Testprobe** | gereinigte Dehydroabietinsäure, Reinheit > 98% | |
| **Panelist** | 5 erfahrene Testpersonen | |
| **Probenvorbereitung** | Dehydroabietinsäure (25µM) gelöst in Ethanol, 24 mg (Stevia-RebA handelsüblich, äquivalent zu 6 % Saccharose) in 100 ml Wasser gelöst, pH Wert wurde durch Zitronensäure eingestellt, Zugabe der Stammlösung bei ca. 20°C, Endkonzentration des Ethanols unter 0,5%, Vergleichsprobe 0,5% Ethanol in Stevia-RebA-Lösung | |
| Bewertung | 25 µM pH neutral | Etwas weniger bitter als Vergleich |
| | | Süßer und runderes Geschmacksprofil als Vergleich |
| | 25 µM pH 3,2 | Unangenehmer Beigeschmack (Bitterkeit und Lakritzaroma) reduziert, |
| | | Süßer und runderes Geschmacksprofil als Vergleich |

### BEISPIEL 13

### Geschmacksmodulierung einer Naringin-Lösung

Die sensorische Beurteilung der Proben wurde von einem Team aus fünf erfahrenen Prüfern durchgeführt. Die Einzelheiten sind den folgenden **Tabelle 11** zu entnehmen:

**Tabelle 11**

| Verkostung Naringin-Lösung | | |
|---|---|---|
| **Testmethode** | deskriptive und diskriminierende Prüfung, "sip and spit"-Methode, Proben verblindet und randomisiert, pH 3,2 und pH neutral | |
| **Testprobe** | gereinigte Dehydroabietinsäure, Reinheit > 98% | |
| **Panelist** | 5 erfahrene Testpersonen | |
| **Probenvorbereitung** | Dehydroabietinsäure (25µM) gelöst in Ethanol, 300 mg Naringin in 100 ml Wasser gelöst, pH Wert wurde durch Zitronensäure eingestellt, Zugabe der Stammlösung bei ca. 20°C, Endkonzentration des Ethanols unter 0,5%, Vergleichsprobe 0,5% Ethanol in Naringin-Lösung | |
| Bewertung | 25 µM pH neutral | Aromatischeres Geschmackprofil als Vergleich Unangenehmer Nachgeschmack (Adstringenz) deutlich reduziert |
| | 25 µM pH 3,2 | unangenehmer Nachgeschmack reduziert, weniger bitter als Vergleich, gleiche Adstringenz wie Vergleich, |

### VERGLEICHSBEISPIEL 14

### Geschmacksmodulierung von Menthol in einem Bonbon

Die sensorische Beurteilung der Proben wurde von einem Team aus fünf erfahrenen Prüfern durchgeführt. Die Einzelheiten sind den folgenden **Tabelle 12** zu entnehmen:

**Tabelle 12**

| Verkostung von Mentholbonbons | | |
|---|---|---|
| **Testmethode** | deskriptive und diskriminierende Prüfung, "sip and spit"-Methode, Proben verblindet und randomisiert, pH 3,2 und pH neutral | |
| **Testprobe** | gereinigte Dehydroabietinsäure, Reinheit > 98% | |
| **Panelist** | 5 erfahrene Testpersonen | |
| **Probenvorbereitung** | Dehydroabietinsäure (25µM) gelöst in Ethanol, 0,3 % Menthol wurden in einem Bonbon formuliert, pH Wert wurde durch Zitronensäure eingestellt, Zugabe der Stammlösung bei > 100°C Endkonzentration des Ethanolsunter 0,5%, Vergleichsproben waren Bonbons ohne Dehydroabietinsäure | |
| Bewertung | 25 µM pH neutral | Süßer und wesentlich geringere Adstringenz als der Vergleich |
| | | Runderes Geschmacksprofil und besseres Aroma als der Vergleich |
| | 25 µM pH 3,2 | Adstringenz vergleichbar, unangenehmer Nachgeschmack reduziert, weniger bitter als der Vergleich |

## Patentansprüche

1. Zubereitungen, umfassend oder bestehend aus
(a) Dehydroabietinsäure, einem Dehydroabietinsäure-Salz und/oder einem Dehydroabietinsäure-haltigen Extrakt und
(b1) mindestens einem von Saccharose verschiedenen Süßstoff ausgewählt sind aus der Gruppe, die gebildet wird von Miraculin, Monellin, Thaumatin, Curculin, Brazzein, Magap, Natriumcyclamat, Acesulfam K, Neohesperidin-Dihydrochalcon, Naringin-Dihydrochalcone, Saccharin Natriumsalz, Aspartam, Superaspartam, Neotam, Alitam, Sucralose, Stevioside, Rebaudioside, Lugduname, Carrelame, Sucrononate, Sucrooctate, Monatin, Hernandulcin, Phyllodulcin, Dihydrochalconglykoside, Glycyrrhizinsäure sowie ihre Derivate und Salze, Extrakte von Lakritz (Glycyrrhiza ssp.), Lippia dulcis Extrakte, Momordica ssp. Extrakte, Mogroside sowie deren Gemische, Saccharin, Steviolglykosiden sowie Stevia-Extrakten und/oder
(b2) mindestens einem in Lebensmitteln eingesetzten anorganischen Salz bzw. Mineralstoff ausgewählt sind aus der Gruppe, die gebildet wird von Kalium- und Magnesiumsalzen und deren Gemischen und/oder
(b3) Limonin,
worin die Zubereitungen die Komponenten (a) in Konzentrationen von 10 bis 250 µM enthalten.

2. Zubereitungen nach Anspruch 1, **dadurch gekennzeichnet, dass** die Extrakte einen Dehydroabietinsäuregehalt von mindestens 20 Gew.-% aufweisen.

3. Nahrungsmittel enthaltend Zubereitungen nach Anspruch 1, wobei die Dehydroabietinsäure oder ein Dehydroabietinsäure-Salz in Konzentrationen von 1 bis 250 µM enthalten ist.

4. Nahrungsmittel nach Anspruch 3, **dadurch gekennzeichnet, dass** es sich um Erfrischungsgetränke oder Heißgetränke oder Instantgetränke handelt.

5. Pharmazeutische Zubereitungen enthaltend Zubereitungen nach Anspruch 1, wobei die Dehydroabietinsäure oder ein Dehydroabietinsäure-Salz in Konzentrationen von 1 bis 250 µM enthalten ist.

6. Pharmazeutische Zubereitungen nach Anspruch 5, **dadurch gekennzeichnet, dass** es sich um Flüssigprodukte handelt.

7. Verfahren zur Maskierung unangenehmer, insbesondere bitterer, adstringierender und/oder Lakritz-artiger Geschmackseindrücke von Miraculin, Monellin, Thaumatin, Curculin, Brazzein, Magap, Natriumcyclamat, Acesulfam K, Neohesperidin-Dihydrochalcon, Naringin-Dihydrochalcone, Saccharin Natriumsalz, Aspartam, Superaspartam, Neotam, Alitam, Sucralose, Stevioside, Rebaudioside, Lugduname, Carrelame, Sucrononate, Sucrooctate, Monatin, Hernandulcin, Phyllodulcin, Dihydrochalconglykoside, Glycyrrhizinsäure sowie ihre Derivate und Salze, Extrakte von Lakritz (Glycyrrhiza ssp.), Lippia dulcis Extrakte, Momordica ssp. Extrakte, Mogroside sowie deren Gemische, Saccharin, Steviolglykosiden sowie Stevia-Extrakten und/oder Kaliumsalzen sowie Nahrungsmitteln oder pharmazeutischen Zubereitungen, die diese Stoffe enthalten, **dadurch gekennzeichnet, dass** man diesen eine Menge an Dehydroabietinsäure, einem Dehydroabietinsäure-Salz und/oder einem Dehydroabietinsäure-haltigen Extrakt zusetzt.

8. Verwendung von Dehydroabietinsäure, einem Dehydroabietinsäure-Salz und/oder einem Dehydroabietinsäure-haltigen Extrakt zur Maskierung unangenehmer, insbesondere bitterer, adstringierender und/oder Lakritz-artiger Geschmackseindrücke von Miraculin, Monellin, Thaumatin, Curculin, Brazzein, Magap, Natriumcyclamat, Acesulfam K, Neohesperidin-Dihydrochalcon, Naringin-Dihydrochalcone, Saccharin Natriumsalz, Aspartam, Superaspartam, Neotam, Alitam, Sucralose, Stevioside, Rebaudioside, Lugduname, Carrelame, Sucrononate, Sucrooctate, Monatin, Hernandulcin, Phyllodulcin, Dihydrochalconglykoside, Glycyrrhizinsäure sowie ihre Derivate und Salze, Extrakte von Lakritz (Glycyrrhiza ssp.), Lippia dulcis Extrakte, Momordica ssp. Extrakte, Mogroside sowie deren Gemische, Saccharin, Steviolglykosiden sowie Stevia-Extrakten und/oder Kaliumsalzen sowie Nahrungsmitteln oder pharmazeutischen Zubereitungen, die diese Stoffe enthalten.

## Claims

1. Compositions, comprising or consisting of
(a) dehydroabietic acid, a salt of dehydroabietic acid and/or an extract comprising dehydroabietic acid, and
(b1) at least one sweetener different from sucrose selected from the group consisting of miraculin, monellin, thaumatin, curculin, brazzein, magap, sodium cyclamate, Acesulfame K, neohesperidin dihydrochalcone, naringin dihydrochalcone, saccharin sodium salt, Aspartame, Super aspartame, Neotame, Alitame, sucralose, stevioside, Rebaudioside, Lugduname, Carrelame, Sucrononate, Sucrooctate, Monatin, Hernandulcin, phyllodulcin, dihydrochalcone glycosides, glycyrrhizic acid and its derivatives and salts, extracts of licorice (Glycyrrhiza ssp.), extracts of Lippia dulcis, extracts of Momordica ssp., Mogroside and its mixtures, saccharin, Steviol glycosides and Stevia extracts and/or
(b2) at least one inorganic salt or minerals used in food preparations selected from the group consisting of potassium salts and magnesium salts and their mixtures and/or
(b3) limonin,
wherein the compositions comprise the components (a) in a concentration of from 10 to 250 µM.

2. Compositions according to claim 1, **characterized in that** the extracts comprise at least 20 wt.% of dehydroabietic acid.

3. Food preparations comprising the compositions according to claim 1, wherein they comprise dehydroabietic acid or a salt of dehydroabietic acid in a concentration of from 1 to 250 µM.

4. Food preparations according to claim 3, **characterized in that** they are soft drinks or hot drinks or instant drinks.

5. Pharmaceutical preparations comprising the compositions according to claim 1, wherein they comprise dehydroabietic acid or a salt of dehydroabietic acid in a concentration of from 1 to 250 µM.

6. Pharmaceutical preparations according to claim 5, **characterized in that**, they are liquid products.

7. A mehtod to mask the unpleasant, especially bitter, astringent and/or licorice-like taste impressions of miraculin, monellin, thaumatin, curculin, brazzein, magap, sodium cyclamate, Acesulfame K, neohesperidin dihydrochalcone, naringin dihydrochalcone, saccharin sodium salt, Aspartame, Super aspartame, Neotame, Alitame, sucralose, stevioside, Rebaudioside, Lugduname, Carrelame, Sucrononate, Sucrooctate, Monatin, Hernandulcin, phyllodulcin, dihydrochalcone glycosides, glycyrrhizic acid and its derivatives and salts, extracts of licorice (Glycyrrhiza ssp.), extracts of Lippia dulcis, extracts of Momordica ssp., Mogroside and its mixtures, saccharin, Steviol glycosides and Stevia extracts and/or potassium salts and food or pharmaceutical preparations comprising these materials, **characterized in that** an amount of dehydroabietic acid, a salt of dehydroabietic acid and/or an extract containing dehydroabietic acid is added.

8. Use of dehydroabietic acid, a salt of dehydroabietic acid and/or an extract containing dehydroabietic acid for masking the unpleasant, especially bitter, astringent and/or licorice-like taste impressions of miraculin, monellin, thaumatin, curculin, brazzein, magap, sodium cyclamate, Acesulfame K, neohesperidin dihydrochalcone, naringin dihydrochalcone, saccharin sodium salt, Aspartame, Super aspartame, Neotame, Alitame, sucralose, stevioside, Rebaudioside, Lugduname, Carrelame, Sucrononate, Sucrooctate, Monatin, Hernandulcin, phyllodulcin, dihydrochalcone glycosides, glycyrrhizic acid and its derivatives and salts, extracts of licorice (Glycyrrhiza ssp.), extracts of Lippia dulcis, extracts of Momordica ssp., Mogroside and its mixtures, saccharin, Steviol glycosides and Stevia extracts and/or potassium salts and food or pharmaceutical preparations comprising these materials.

## Revendications

1. Préparations, comprenant ou constituées de
(a) acide déhydroabiétique, un sel d'acide déhydroabiétique et/ou un extrait contenant de l'acide déhydroabiétique et
(b1) au moins un édulcorant différent du saccharose choisi dans le groupe qui est formé par la miraculine, la monelline, la thaumatine, la curculine, la brazzéine, le magap, le cyclamate de sodium, l'acésulfame K, la néohespéridine-dihydrochalcone, la naringine-dihydrochalcone, le sel de sodium de la saccharine, l'aspartame, le superaspartame, le néotame, l'alitame, le sucralose, le stévioside, le rébaudioside, le lugduname, le carrélame, le sucrononate, le sucrooctate, la monatine, l'hernandulcine, la phyllodulcine, le dihydrochalconeglycoside, l'acide glycyrrhizique ainsi que leurs dérivés et leurs sels, les extraits de réglisse (Glycyrrhiza ssp.), les extraits de Lippia dulcis, les extraits de Momordica ssp., les mogrosides ainsi que leurs mélanges, la saccharine, les glycosides de stéviol ainsi que les extraits de stévia et/ou
(b2) au moins un sel inorganique respectivement une substance minérale utilisé(e) dans des denrées alimentaires, choisi(e) dans le groupe formé par les sels de potassium et de magnésium et leurs mélanges et/ou
(b3) la limonine,
les préparations contenant les composants (a) en des concentrations de 10 à 250 µM.

2. Préparations selon la revendication 1, **caractérisées en ce que** les extraits présentent une teneur en acide déhydroabiétique d'au moins 20 % en poids.

3. Produit alimentaire contenant des préparations selon la revendication 1, l'acide déhydroabiétique ou un sel d'acide déhydroabiétique étant contenu en des concentrations de 1 à 250 µM.

4. Produit alimentaire selon la revendication 3, **caractérisé en ce qu'**il s'agit de boissons rafraîchissantes ou de boissons chaudes ou de boissons instantanées.

5. Préparations pharmaceutiques contenant des préparations selon la revendication 1, l'acide déhydroabiétique ou un sel d'acide déhydroabiétique étant contenu en des concentrations de 1 à 250 µM.

6. Préparations pharmaceutiques selon la revendication 5, **caractérisées en ce qu'**il s'agit de produits liquides.

7. Procédé pour le masquage d'impressions gustatives désagréables, en particulier amères, astringentes et/ou de type réglisse de la miraculine, de la monelline, de la thaumatine, de la curculine, de la brazzéine, du magap, du cyclamate de sodium, de l'acésulfame K, de la néohespéridine-dihydrochalcone, de la naringine-dihydrochalcone, du sel de sodium de la saccharine, de l'aspartame, du superaspartame, du néotame, de l'alitame, du sucralose, du stévioside, du rébaudioside, du lugduname, du carrélame, du sucrononate, du sucrooctate, de la monatine, de l'hernandulcine, de la phyllodulcine, du dihydrochalconeglycoside, de l'acide glycyrrhizique ainsi que de leurs dérivés et de leurs sels, des extraits de réglisse (Glycyrrhiza ssp.), des extraits de Lippia dulcis, des extraits de Momordica ssp., des mogrosides ainsi que de leurs mélanges, de la saccharine, des glycosides de stéviol ainsi que des extraits de stévia et/ou des sels de potassium ainsi que de produits alimentaires ou de préparations pharmaceutiques qui contiennent ces matières, **caractérisé en ce qu'**on ajoute à ceux-ci/celles-ci une quantité d'acide déhydroabiétique, d'un sel d'acide déhydroabiétique et/ou d'un extrait contenant de l'acide déhydroabiétique.

8. Utilisation d'acide déhydroabiétique, d'un sel d'acide déhydroabiétique et/ou d'un extrait contenant de l'acide déhydroabiétique pour le masquage d'impressions gustatives désagréables, en particulier amères, astringentes et/ou de type réglisse de la miraculine, de la monelline, de la thaumatine, de la curculine, de la brazzéine, du magap, du cyclamate de sodium, de l'acésulfame K, de la néohespéridine-dihydrochalcone, de la naringine-dihydrochalcone, du sel de sodium de la saccharine, de l'aspartame, du superaspartame, du néotame, de l'alitame, du sucralose, du stévioside, du rébaudioside, du lugduname, du carrélame, du sucrononate, du sucrooctate, de la monatine, de l'hernandulcine, de la phyllodulcine, du dihydrochalconeglycoside, de l'acide glycyrrhizique ainsi que de leurs dérivés et de leurs sels, des extraits de réglisse (Glycyrrhiza ssp.), des extraits de Lippia dulcis, des extraits de Momordica ssp., des mogrosides ainsi que de leurs mélanges, de la saccharine, des glycosides de stéviol ainsi que des extraits de stévia et/ou des sels de potassium ainsi que de produits alimentaires ou de préparations pharmaceutiques qui contiennent ces matières.
